# EUROPEAN PATENT APPLICATION

(11) **EP 4 309 662 A1**
(43) Date of publication of application: **24.01.2024**
(21) Application number: 22185855.8
(22) Date of filing: 19.07.2022
(51) Int. Cl.: A61K 35/74, A23L 33/135, A61P 3/08, A61P 3/10, C12N 1/20

(54) **MICROORGANISMS CONVERTING INOSITOL TO BUTYRATE**

(71) Applicant: Enbiosis Biotechnology Limited, Leicester LE1 1LB (GB)
(72) Inventor: Nalbantoglu, Özkan Ufuk, Izmir (TR); Özkan, Ömer, Istanbul (TR); Gündogdu, Aycan, Ankara (TR)
(74) Representative: Sach, Greg Robert

(57) **Abstract**

The present invention relates to the field of prevention and/or treatment of metabolic disorders such as obesity, metabolic syndrome, type-2 diabetes, dyslipidemia and insulin resistance.

## Description

### TECHNICAL FIELD

The present invention relates to the field of metabolic health and to prevention and/or treatment of metabolic disorders such as obesity, metabolic syndrome, type-2 diabetes, dyslipidemia and insulin resistance.

### BACKGROUND ART

Butyrate is a short-chain fatty acid synthesized in the human gut microbiome, usually as a result of carbohydrate conversions. Short-chain fatty acids are mainly the end products of the fermentation of undigested sugars and dietary fiber by the intestinal bacterial flora. Short chain fatty acids are organic fatty acids with 1 to 6 carbon atoms. Short-chain fatty acids with different carbon chain lengths (acetate (C2), propionate (C3), butyrate (C4), valerate (C5) and caproate (C6)) are produced in varying amounts depending on the diet and the composition of the intestinal microbiota. Acetate (C2), propionate (C3) and butyrate (C4) are the most important short-chain fatty acids in mammalian physiology. Propionic and butyric acid are produced as a result of the activity of special bacterial groups. Acetate, which constitutes the majority of short-chain fatty acids, is produced by many bacterial groups. In addition, this acetate produced is used by intestinal bacteria for the production of butyrate. Known mechanisms of butyrate biosynthesis are generally explained by the fermentation of complex sugars/fibers by bacteria colonized within the gastrointestinal tract (McIntyre et al., 1993). Due to this bioconversion, human metabolism is exposed to significant concentrations of butyrate postbiotics originating from the gut microbiome. Butyrate is known to be closely related to human health. It is known that the metabolic syndrome associated with obesity is caused by butyrate deficiency, and external butyrate supplementation is effective in the treatment of the disease (Gao et al. 2019, Bridgeman et al., 2020). On the other hand, butyrate, which is known to have a positive effect on insulin sensitivity (Gao et al., 2009), is also seen as a potential compound in the treatment of type-2 diabetes (Arora and Tremaroli, 2021).

However, it has also been observed that myo-inositol compounds, which are found in high amounts in the human body and can be obtained from plant food sources, and can also be converted from glucose by the human body, have a lowering effect on post-prandial glucose levels (Ortmayer et al., 1993, Croze and Soulage, 2013), but the mechanism by which this effect occurs is not yet known.

Butyrate or butyric acid is a very beneficial metabolite for health, and it is a nutrient that cannot be easily taken externally through food. Certain bacterial groups in the intestine can produce butyrate from the fiber. These bacteria are typically present in healthy people, however, our daily consumption of fiber foods, in general, is extremely low and not regular. Therefore, even if these bacteria are present or taken externally as a probiotic supplement, they do not produce butyrate if fiber is not consumed daily.

Besides these bacteria, another microorganism that cannot be propagated in the laboratory (due to the unknown propagation conditions) also produces butyric acid from sugar. However, this bacterium is not present in every person.

Although microorganisms with pathways that produce short-chain fatty acids such as propionate or acetate from myo-inositol have been identified, an organism that produces butyrate from this substrate was not yet available. The efficacy of organisms with such pathway in the prevention/treatment of metabolic disorders has not been previously observed. The present invention relates to, based on this observation, to the use of these organisms for the prevention/treatment thereof.

As a result, due to the reasons explained above and since the existing solutions are not sufficient, there is a need in a technical development in this field.

### BRIEF DESCRIPTION OF THE INVENTION

The solution to the afore-mentioned objective technical problem underlying the invention is described in the claims.

The present invention relates to microorganisms that carry a specific gene cluster that converts inositol to butyrate thereby converting inositol molecules into butyric acid, salt or ester thereof and/or the uses thereof to prevent or treat metabolic syndrome, insulin resistance and/or associated health conditions.

Specifically, the microorganism has the capacity to convert inositol into butyric acid and its derivatives through genes that metabolize inositol and synthesize butyrate. Said inositol may be in the form of myo-inositol or chiro-inositol, but preferably myo-inositol.

If the aforementioned microorganism strains are provided to a human in sufficient amounts by topical or oral route, it is expected to survive and colonize, at least temporarily, in the gastrointestinal tract or other body parts of said human. In this colonization situation, it is expected that the increase in butyrate production in the human body *in situ* will have a positive effect on human health, and have an effect in the prevention or treatment of disorders such as metabolic syndrome, insulin resistance, obesity, and type-2 diabetes.

In particular, the present invention relates to a microorganism comprising one or more gene(s) that encode proteins of a metabolic pathway that can convert inositol compounds to butyric acid, salt or ester thereof for use in the prevention and/or treatment of metabolic disorders, **characterized in that** the at least one or more genes encoding the metabolic pathway proteins are selected from the group consisting of myo-inositol 2-dehydrogenase, 2-keto-myo-inositol dehydratase, 3D-(3,5/4)-trihydroxycyclohexane-1,2-dione hydrolase, 5-deoxyglucuronate isomerase, 5-dehydro-2-deoxygluconokinase, 6-phospho-5-dehydro-2-deoxy-D-gluconate aldolase, Enoyl-CoA hydratase/carnithine racemase, butyryl-CoA dehydrogenase, Electron transfer flavoproteins and butyryl CoA:acetate CoA transferase.

Preferably, the metabolic syndrome is obesity, metabolic syndrome, type-2 diabetes, dyslipidemia or insulin resistance.

In another preferred embodiment, the microorganism is bacteria, yeast of fungi, more preferably the bacteria, yeast or fungi is produced synthetically by genetic engineering and/or the one or more gene(s) of the metabolic pathway is transferred in vitro into the bacteria, yeast or fungi. In another preferred embodiment, the bacteria is an isolate isolated from the human intestine.

Preferably, the one or more genes encoding protein(s) of the metabolic pathway has at least 70 % sequence identity with a sequence selected from the group consisting of Seq Id No. 1, 3, 5, 7, 9, 11, 13, 15, 17, 19, 21, 23, 25 and 27.

In one aspect of the invention, the microorganism is in a physiologically acceptable carrier composition.

Preferably, the composition is a food composition, preferably a dairy product or a fermented dairy product, preferably a yogurt or a yogurt drink, or a pharmaceutical composition, preferably in solid dosage form, preferably in the form of a capsule, a tablet or a powder.

Preferably, the microorganism may be present in the composition in lyophilized or microencapsulated form and/or the composition may be as a probiotic for administration.

In another aspect of the invention, the invention relates to the use of the microorganism or composition according to the present invention for protecting, restoring and/or improving metabolic health. This may include maintaining healthy body composition, maintaining healthy glucose or insulin metabolism, treating and/or preventing overweight and/or maintaining a healthy fat (or lipid) metabolism.

In a further aspect, the invention relates to a method of treatment of metabolic diseases, such as obesity, metabolic syndrome, type 2 diabetes, dyslipidemia or insulin resistance for a subject in need with the microorganism of composition according to the present invention or a method for protecting, restoring and/or improving metabolic health. The mircroorganism or composition is administered to the subject or patient in need of.

The present inventors examined the relationship between mean glucose levels and elements of the gut microbiome in studies conducted on subjects whose blood glucose levels were monitored 24 hours a day for 10 days with continuous glucometer sensors. Accordingly, shotgun metagenome new generation sequencing data obtained as a result of total DNA isolation of fecal samples taken before the experiment, as well as a total of 240 hours of continuous blood glucose data per person, were produced by blood glucose measurements made every 15 seconds for all of the subjects. Characterization of each individual's gut microbiome at the metabolic pathway level was performed and it was found that the mean blood glucose levels of individuals with the pathway that converts myo-inositol to butyrate as mentioned in the invention were significantly lower than those without this pathway (p < 10⁻⁵, Mann-Whitney U-test). Similarly, it was observed that the body mass indexes of individuals with the aforementioned pathway were lower than those without this (p < 10⁻⁶, Mann-Whitney U-test). Finally, it was reported that the HDL cholesterol levels of the same group were higher than those without the pathway (p < 10⁻⁵, Mann-Whitney U-test). In this case, it is hypothesized that the significant difference in the general metabolic health markers of individuals with microorganisms capable of converting myo-inositol to butyrate is due to the metabolic function in question, and it is suggested that if microorganisms with this pathway are provided to individuals in a functional way, they can be probiotic/biotherapeutic agent candidates to be used for the prevention and/or treatment of metabolic disorders such as obesity, metabolic syndrome, type-2 diabetes, dyslipidemia and insulin resistance.

In the present invention the metabolic pathway that is not found in every person and that produces butyric acid from sugar we take through every meal daily is turned into a probiotic that it is possible to produce in the laboratory.

### BRIEF DESCRIPTION OF THE FIGURES

Figure 1: The weekly average postprandial blood sugar spikes of the observed subgroups. People having the myo-inositol to butyrate transformation pathways in their gut microbiome experience statistically significantly lower number of weekly spikes compared to the people lacking that pathway.
Figure 2. Baseline HbA1C (%) levels of the observed subgroups. People having the myo-inositol to butyrate transformation pathways in their gut microbiome experience statistically significantly lower blood sugar levels compared to the people lacking that pathway.

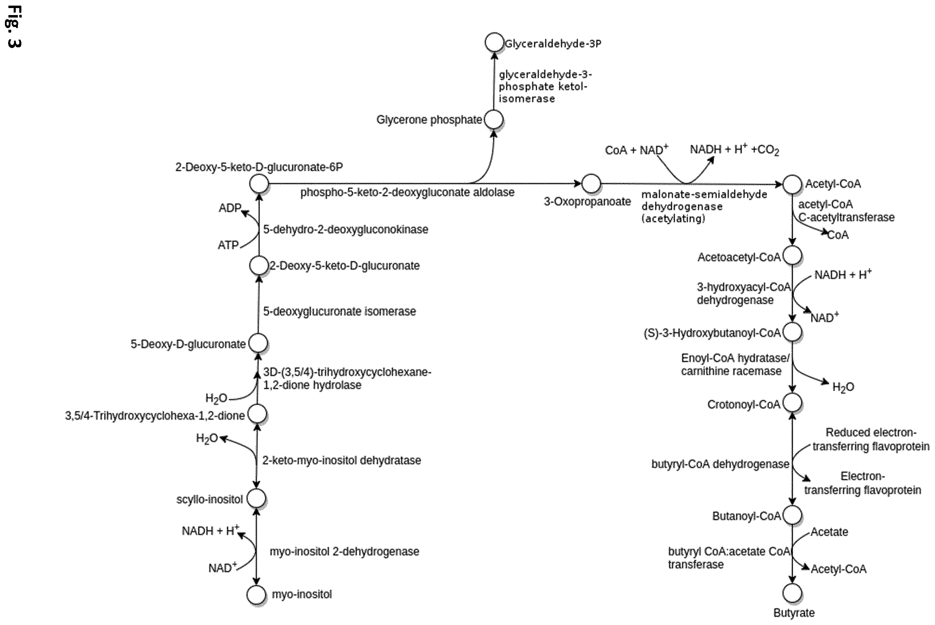

Figure 3. Myo-inositol butyrate pathway. The figure provides a schematic view of the myo-inositol butyrate transformation pathway.

### DETAILED DESCRIPTION OF THE INVENTION

In this detailed description, the preferred alternatives of microorganisms belonging to the metabolic pathways that produce butyrate from myo-inositol, developed as the subject of the invention, are explained only for a better understanding of the subject without any limitation.

### Microorganism

Primarily, the invention is directed to a microorganism comprising a gene cluster that encodes an inositol-butyrate pathway which enables the microorganism to convert inositol to butyric acid or a salt or ester thereof, preferably under anaerobic conditions. The microorganism may be an isolate belonging to the bacterial, archaeal, or fungal kingdoms.

The gene cluster of the pathway that converts inositol to butyrate comprises at least one of the genes encoding the following proteins: myo-inositol 2-dehydrogenase, 2-keto-myo-inositol dehydratase, 3D-(3,5/4)-trihydroxycyclohexane-1,2-dione hydrolase, 5-deoxyglucuronate isomerase, 5-dehydro-2-deoxygluconokinase, 6-phospho-5-dehydro-2-deoxy-D-gluconate aldolase, Enoyl-CoA hydratase/carnithine racemase, butyryl-CoA dehydrogenase, Electron transfer flavoproteins and butyryl CoA:acetate CoA transferase.

The microorganism in question may be a natural bacterial isolate isolated from the human intestine or an environmental sample, or it may be a genetically modified bacterium, archaea or fungus, in which one or more of the above-mentioned genes have been transferred in vitro.

### Genes:

The gene encoding the butyryl-CoA dehydrogenase has at least 70, 80, 90, 95, 99, 100% identity with Sequence ID No. 1. The respective amino acid sequence is shown in Sequence ID. No. 2.

The gene encoding the electron transfer flavoprotein has at least 70, 80, 90, 95, 99, 100% identity with Sequence ID No. 3 or Sequence ID No. 5 or Sequence ID No. 7 or Sequence ID No. 9. The respective amino acid sequences are shown in Sequences ID. No. 4, 6, 8 and 10.

The gene encoding the butyryl CoA:acetate CoA transferase has at least 70, 80, 90, 95, 99, 100% identity with Sequence ID No. 11. The respective amino acid sequence is shown in Sequence ID. No. 12.

The gene encoding the enoyl-CoA hydratase/carnithine racemase has at least 70, 80, 90, 95, 99, 100% identity with Sequence ID No. 13. The respective amino acid sequence is shown in Sequence ID. No. 14.

The gene encoding the myo-inositol 2-dehydrogenase has at least 70, 80, 90, 95, 99, 100% identity with Sequence ID No. 15. The respective amino acid sequence is shown in Sequence ID. No. 16.

The gene encoding the 2-keto-myo-inositol dehydratase has at least 70, 80, 90, 95, 99, 100% identity with Sequence ID No. 17 or identity with Sequence ID No. 19. The respective amino acid sequence are shown in Sequence ID. Nos. 18 and 20.

The gene encoding the 5-dehydro-2-deoxygluconokinase has at least 70, 80, 90, 95, 99, 100% identity with Sequence ID No. 21. The respective amino acid sequence is shown in Sequence ID. No. 22.

The gene encoding the 3D-(3,5/4)-trihydroxycyclohexane-1,2-dione hydrolase has at least 70, 80, 90, 95, 99, 100% identity with Sequence ID No. 23. The respective amino acid sequence is shown in Sequence ID. No. 24.

The gene encoding the 5-deoxyglucuronate isomerase has at least 70, 80, 90, 95, 99, 100% identity with Sequence ID No. 25. The respective amino acid sequence in shown in Sequence ID. No. 26.

The gene encoding the 6-phospho-5-dehydro-2-deoxy-D-gluconate aldolase has at least 70, 80, 90, 95, 99, 100% identity with Sequence ID No. 27. The respective amino acid sequence in shown in Sequence ID. No. 28.

### Composition:

The present invention also includes a composition comprising a bacterium according to the present disclosure and optionally a physiologically acceptable carrier. The physiologically acceptable carrier may be any suitable carrier for keeping the microorganism with said properties alive until it is consumed by a target organism (e.g., human or animal). For example, examples of acceptable carriers suitable for this purpose include any of the well known physiological or pharmaceutical carriers, buffers, and intermediates. The selection of an appropriate physiological or pharmaceutical carrier may vary depending on the intended mode of administration of the composition (e.g., oral) and the intended form of the composition (e.g., beverage, yogurt, powder, capsules, etc.) as discussed herein.

The composition containing said microorganisms may be in the form of food (food), feed, food formulation, food supplement composition or pharmaceutical composition.

Preferably said composition is a food or food supplement composition. The food or food supplement composition may be selected from the group consisting of a liquid, liquid beverage (including milk beverage and fermented beverage), yogurt, cheese, gel, gelatin, gelatin capsule, powder, paste, pressed tablet, and gel cap. The food or food supplement composition may be a dairy product, preferably a fermented dairy product, preferably a yogurt or a yogurt drink.

Preferably, said composition may be a probiotic composition. Such probiotic composition may include (isolated) bacteria, fungi, or a strain derived therefrom, as discussed herein. Preferably, the composition may further comprise one or more additional beneficial isolated strains of intestinal microorganism.

Preferably said composition may contain one or more prebiotic ingredients, any prebiotic ingredient suitable for increasing the activity of the microorganism and/or stimulating its growth. Examples of suitable prebiotic ingredients include fibers such as inulin, pectin, and resistant starch, as well as inositol, cellobiose, maltose, mannose, salicin, trehalose, amygdalin, arabinose, melibiose, sorbitol, rhamnose and/or xylose.

Preferably said composition comprises a (myo)inositol rich source and/or (myo)inositol. For example, it may be advantageous to add (myo)inositol rich source and/or (myo)inositol to the composition to further stimulate the production of butyric acid/butyrate and/or propionic acid/propionate or a derivative thereof, as taught herein.

Said microorganism may be contained in lyophilized form, microencapsulated form (see for example, Solanki et al., BioMed Res. Int. 2013, Article ID 620719), or any other form that protects the viability and activity of the microorganism species.

Preferably, said composition may be a pharmaceutical composition. The pharmaceutical composition may be produced for use as a supplement. A pharmaceutical composition will generally contain a pharmaceutical carrier in addition to the microorganism as mentioned herein. The carrier is preferably an inactive carrier. The preferred form depends on the intended mode of administration and (therapeutic) application. A pharmaceutical carrier may be any compatible, non-toxic substance suitable for delivering the organism mentioned herein into the body of a subject. For example, sterile water or inactive solids can be used as a carrier, often complemented by a pharmaceutically acceptable adjuvant, buffering agent, dispersing agent, and the like. The pharmaceutical composition may be in liquid form (e.g., a stabilized bacterial suspension of said microorganism) or in solid form (e.g., powder of said lyophilized microorganism). When said microorganism is lyophilized, a cryoprotectant such as lactose, trehalose or glycogen can be used. For example, for oral administration, said microorganism may be administered in solid dosage forms such as capsules, tablets and powders containing lyophilized microorganism, or in liquid dosage forms such as syrups and suspensions. Said microorganism may be carried, for example, in lyophilized form, in capsules such as gelatin capsules, in the form of inactive ingredients and, for example, glucose, lactose, sucrose, mannitol, starch, cellulose or cellulose derivatives, magnesium stearate, stearic acid, sodium saccharin, talc, magnesium carbonate and the like.

With the invention, it is intended to be used for the protection, restoration and/or improvement of gastrointestinal system health in general and/or for the prevention and/or treatment of conditions or diseases such as obesity, metabolic diseases. This includes the metabolic syndrome associated with obesity and insulin resistance or complications related to insulin resistance, such as dyslipidemia and type 2 diabetes mellitus. It also includes embodiments of the present invention for the purpose of protecting, restoring and/or improving gastrointestinal system health in general and/or preventing and/or treating obesity, metabolic diseases such as metabolic syndrome and insulin resistance, and conditions or diseases related to insulin resistance. It is intended herein to observe the expected efficacy by administering an effective amount of said microorganism to said subject by said means.

The following example that follows further illustrates the invention but should not be construed to limit the scope of the invention in any way.

### EXAMPLE

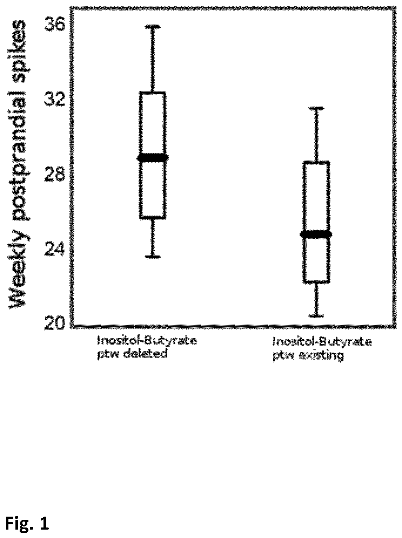

The discovery of phenotypic effects of putative inositol-butyrate conversion molecular pathways was conducted via an observational study. The observation cohort consisted of 100 individuals under continuous blood sugar monitoring using continuous glucometers (Dexcom, San Diego, CA, USA). The blood sugar levels of the individuals were recorded for 10 days with 15 seconds sampling periods, creating 240 hours/person continuous blood sugar measurements. Stool samples of each participant were collected using appropriate fecal sampling techniques at the baseline. At the same time window, biochemical monitoring of each individual from peripheral blood was conducted. The fecal samples were subject to total DNA isolation. In order to evaluate the gut microbiome, shotgun metagenome sequencing was performed. Raw DNA sequencing data were analyzed using bioinformatic techniques. Each metagenome sample were mapped to reference genomes of known gut microbes and the relative coverage of each genomic regions were estimated. Characterization of the metabolic pathways were achieved by using KEGG pathways database. It was observed that a genomic region harboring myo-inositol to butyrate transformation pathway defined here is significantly altering between individuals with varying glycemic responses. This characterization is defined in two different subgroups. Firstly, the subjects were observed based on their post-prandial glycemic responses. A post-prandial spike was defined as overshooting the baseline estimated blood sugar level (mmol/L) by 100% within 2 hours of a meal consumption. The weekly average postprandial spike numbers of each individual was compared based on harboring the defined inositol-butyrate conversion molecular pathway. The observation cohort was divided into two subgroups which are the inositol-butyrate conversion molecular pathway deleted group (the genomic region containing the pathway has less than 5% coverage of the rest of the genome) and the inositol-butyrate conversion molecular pathway harboring group. It was observed that the pathway deletion group experiences more number of weekly postprandial sugar spikes compared to the people having a gut microbiome that contains the inositol-butyrate conversion pathway (p < 10-5, Mann-Whitney U-test) (Figure 1)

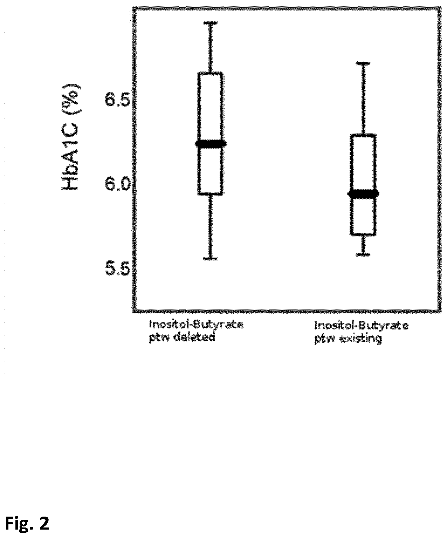

Secondly, the stable blood sugar levels of these individuals, based on HbA1C (%) measurements were compared, and a similar trend of lower HbA1C levels in inositol-butyrate conversion pathway group was determined (p < 10-6, Mann-Whitney U-test) (Figure 2).

Similar observations were reperated in body-mass index comparison (lower in inositol-butyrate conversion pathway group, p < 10-6, Mann-Whitney U-test) and in high-density lipoprotein (HDL) cholestreol levels (lower in inositol-butyrate conversion pathway group, p < 10-5, Mann-Whitney U-test).

Based on the experiments conducted in this cohort, it is hypothesized that people harboring microorganisms that are able to convert myo-inositol into butyrate in their gut microbiome are in a trend of having healthier metabolic indicators. In case that organisms having that function are administered in an appropriate way, they can provide health benefits to the recepient experiencing metabolic disorders such as obesity, metabolic syndrome, type-2 diabetes, dyslipidemia, and insulin resistance. Furthermore, organisms with that property can be positioned as probiotics/biotherapeutics for prevention and treatment of metabolic disorders.

### References:

McIntyre A, Gibson PR, Young GP. Butyrate production from dietary fiber and protection against large bowel cancer in a rat model. Gout. Mar 1993 1;34(3):386-91.
Gao F, Lv YW, Long J, Chen JM, He JM, Ruan XZ, Zhu HB. Butyrate improves the metabolic disorder and gut microbiome dysbiosis in mice induced by a high-fat diet. Frontiers in pharmacology. 2019:1040.
Bridgeman SC, Northrop W, Melton PE, Ellison GC, Newsholme P, Mamotte CD. Butyrate generated by gut microbiota and its therapeutic role in metabolic syndrome. Pharmacological research. 2020 Oct 1;160:105174.
Gao Z, Yin J, Zhang J, Ward RE, Martin RJ, Lefevre M, Cefalu WT, Ye J. Butyrate improves insulin sensitivity and increases energy expenditure in mice. Diabetes. 2009 Jul 1;58(7):1509-17.
Arora T, Tremaroli V. Therapeutic potential of butyrate for treatment of type 2 diabetes. Frontiers in endocrinology. Oct 2021 19:1313.
Ortmeyer HK, Huang LC, Zhang L, Hansen BC, Larner JO. Chiroinositol deficiency and insulin resistance. II. Acute effects of D-chiroinositol administration in streptozotocin-diabetic rats, normal rats given a glucose load, and spontaneously insulin-resistant rhesus monkeys. Endocrinology. 1993 Feb 1;132(2):646-51.
Croze ML, Soulage CO. Potential role and therapeutic interests of myo-inositol in metabolic diseases. Biochimie. 2013 Oct 1;95(10):1811-27.
Solanki HK, Pawar DD, Shah DA, Prajapati VD, Jani GK, Mulla AM, Thakar PM. Development of microencapsulation delivery system for long-term preservation of probiotics as biotherapeutics agent. BioMed research international. Jan 2013 1; 2013.

## Claims

1. A microorganism comprising one or more gene(s) that encode proteins of a metabolic pathway that can convert inositol compounds to butyric acid, salt or ester thereof for use in the prevention and/or treatment of metabolic disorders, **characterized in that** the at least one or more genes encoding the metabolic pathway proteins are selected from the group consisting of myo-inositol 2-dehydrogenase, 2-keto-myo-inositol dehydratase, 3D-(3,5/4)-trihydroxycyclohexane-1,2-dione hydrolase, 5-deoxyglucuronate isomerase, 5-dehydro-2-deoxygluconokinase, 6-phospho-5-dehydro-2-deoxy-D-gluconate aldolase, Enoyl-CoA hydratase/carnithine racemase, butyryl-CoA dehydrogenase, Electron transfer flavoproteins and butyryl CoA:acetate CoA transferase.

2. The microorganism of claim 1, wherein the metabolic syndrome is obesity, metabolic syndrome, type-2 diabetes, dyslipidemia or insulin resistance.

3. The microorganism of claim 1 or 2, wherein the microorganism is bacteria, yeast or fungi.

4. The microorganism of claim 3, wherein the bacteria, yeast or fungi is produced synthetically by genetic engineering.

5. The microorganism of claim 3 or 4, wherein the one or more gene(s) is transferred in vitro into the bacteria, yeast or fungi.

6. The microorganism of claim 3, wherein the bacteria is an isolate isolated from the human intestine.

7. The microorganism of any one of the preceding claims, wherein the one or more genes encoding protein(s) of the metabolic pathway has at least 70 % sequence identity with a sequence selected from the group consisting of Seq Id No. 1, 3, 5, 7, 9, 11, 13, 15, 17, 19, 21, 23, 25 and 27.

8. The microorganism of any one of the preceding claims, wherein it is in a physiologically acceptable carrier composition.

9. The composition according to claim 8, wherein it is a food composition, preferably a dairy product or a fermented dairy product, preferably a yogurt or a yogurt drink.

10. The composition according to claim 8, wherein it is a pharmaceutical composition, preferably in solid dosage form, preferably in the form of a capsule, a tablet or a powder.

11. The composition according to any one of claims 8-10, wherein the microorganism is present in the composition in lyophilized or microencapsulated form.

12. The composition according to any of the preceding claims as a probiotic.
